# EUROPEAN PATENT APPLICATION

(11) **EP 2 915 561 A1**
(43) Date of publication of application: **09.09.2015**
(21) Application number: 15153628.1
(22) Date of filing: 03.02.2015
(51) Int. Cl.: A61N 1/39

(54) **Portable defibrillation device, mobile terminal, and operating method of the mobile terminal**

(30) Priority: 04.03.2014 KR 20140025492
(71) Applicant: Nousco, Inc., Seoul (KR)
(72) Inventor: Baek, Chang Woo, Seoul (KR); Kim, Sung Hyun, Seoul (KR); Jeon, Il Hyun, Seoul (KR)
(74) Representative: Tischner, Oliver

(57) **Abstract**

Provided are a portable defibrillation device, a mobile terminal, and an operating method of the mobile terminal, in which the portable defibrillation device may initiate charging of an energy storage or output an electrical shock using the energy storage in response to a control signal to be received from the mobile terminal.

## Description

### BACKGROUND

### 1. Field of the Invention

The present invention relates to a portable defibrillation device, a mobile terminal, and an operating method of the mobile terminal.

### 2. Description of the Related Art

Development of medical technology has contributed to a longer human lifespan. Nevertheless, a mortality rate from sudden death is relatively high. A great number of sudden death cases relate closely to a cardiac disorder, for example, a myocardial infarction and a cardiac arrest.

Most cases of the cardiac arrest may be treated when a basic cardiopulmonary resuscitation (CPR) and an electrical shock are provided within a few minutes of an occurrence of a cardiac standstill. However, most cases of the cardiac disorder such as the cardiac arrest may occur unexpectedly. In addition, carrying a defibrillation device that applies an electrical shock to a heart may not be easy for general people.

Thus, there is a desire for a defibrillation device that is portable and convenient to use for anyone in an emergency.

### SUMMARY

According to an aspect of the present invention, there is provided a portable defibrillation device including a communication unit configured to communicate with a mobile terminal, a charging unit configured to charge an energy storage for defibrillation, an electrode unit configured to output an electrical shock using the energy storage, and a control unit configured to control the charging unit to initiate the charging of the energy storage in response to a first signal received through the communication unit, and control the charging unit to allow the electrical shock to be output in response to a second signal received through the communication unit. The first signal may be automatically generated based on whether the electrical shock is required for the defibrillation.

The control unit may control the communication unit to allow a biosignal detected through the electrode unit to be transmitted to the mobile terminal. At least one of the first signal and the second signal may be generated based on the biosignal.

The first signal may be generated based on at least one of an output of an acceleration sensor included in the mobile terminal and an output of a wearable device worn by a user.

The charging unit may charge the energy storage using a power source of the mobile terminal. The portable defibrillation device may further include a power source electrically connected to the charging unit. The control unit may control the charging unit to charge the energy storage using at least one of the power source of the portable defibrillation device and the power source of the mobile terminal based on the first signal.

The first signal may be further generated based on at least one of an amount of power remaining in the power source of the mobile terminal, an output characteristic of the power source of the mobile terminal, an amount of power remaining in the power source of the portable defibrillation device, and an output characteristic of the power source of the portable defibrillation device.

The control unit may control the charging unit to charge the energy storage using the power source of the mobile terminal when information included in the first signal indicates a first charging mode. The control unit may control the charging unit to charge the energy storage using the power source of the portable defibrillation device when the information included in the first signal indicates a second charging mode. The control unit may control the charging unit to charge the energy storage using the power source of the portable defibrillation device and the power source of the mobile terminal when the information included in the first signal indicates a third charging mode.

The power source of the portable defibrillation device may supply auxiliary power to the mobile terminal. In addition, the portable defibrillation device may further include a combining unit combined to the mobile terminal.

According to another aspect of the present invention, there is provided a mobile terminal including a communication unit configured to communicate with a portable defibrillation device, and a control unit configured to generate a first signal based on a determination that an electrical shock is required for defibrillation, control the communication unit to allow the first signal to be transmitted to the portable defibrillation device, generate a second signal based on a determination that a condition for the electrical shock is satisfied, and control the communication unit to allow the second signal to be transmitted to the portable defibrillation device.

The communication unit may receive information associated with a biosignal of a user, and the control unit may determine whether the electrical shock is required for the defibrillation based on the information associated with the biosignal of the user.

The mobile terminal may further include a reception unit configured to receive information associated with a biosignal of the user from a wearable device worn by the user, and the control unit may determine whether the electrical shock is required for the defibrillation based on the information associated with the biosignal of the user.

The mobile terminal may further include a network communication unit configured to communicate with a server connected through a network. The control unit may control the network communication unit to allow the information associated with the biosignal of the user to be transmitted to the server, and determine whether the electrical shock is required for the defibrillation based on a signal received through the network communication unit.

The mobile terminal may further include an acceleration sensor configured to measure an acceleration of the mobile terminal, and the control unit may determine whether the electrical shock is required for the defibrillation based on an output of the acceleration sensor.

The communication unit may receive information as to whether electrode pads included in the portable defibrillation device are attached to the user, and the control unit may determine whether the condition for the electrical shock is satisfied based on the information as to whether the electrode pads are attached to the user.

The communication unit may receive information associated with a biosignal detected by the electrode pads included in the portable defibrillation device, and the control unit may calculate a timing for the electrical shock based on the information associated with the biosignal.

The control unit may determine a charging mode of an energy storage for the defibrillation based on at least one of an amount of power remaining in a power source of the mobile terminal, an output characteristic of the power source of the mobile terminal, an amount of power remaining in a power source of the portable defibrillation device, and an output characteristic of the power source of the portable defibrillation device.

The charging mode of the energy storage may include a first charging mode using the power source of the mobile terminal, a second charging mode using the power source of the portable defibrillation device, and a third charging mode using the power source of the mobile terminal and the power source of the portable defibrillation device.

The mobile terminal may further include a combining unit combined to the portable defibrillation device.

According to still another aspect of the present invention, there is provided a portable defibrillation device including a communication unit configured to communicate with a mobile terminal, a charging unit configured to charge an energy storage for defibrillation, an electrode unit configured to output an electrical shock using the energy storage, a power source electrically connected to the charging unit, and a control unit configured to control the charging unit to charge the energy storage using at least one of the power source of the portable defibrillation device and a power source of the mobile terminal based on a first signal to be received through the communication unit.

The first signal may be generated based on at least one of an amount of power remaining in the power source of the mobile terminal, an output characteristic of the power source of the mobile terminal, an amount of power remaining in the power source of the portable defibrillation device, and an output characteristic of the power source of the portable defibrillation device.

The control unit may control the charging unit to charge the energy storage using the power source of the mobile terminal when information included in the first signal indicates a first charging mode. The control unit may control the charging unit to charge the energy storage using the power source of the portable defibrillation device when the information included in the first signal indicates a second charging mode. The control unit may control the charging unit to charge the energy storage using the power source of the portable defibrillation device and the power source of the mobile terminal when the information included in the first signal indicates a third charging mode.

The control unit may control the charging unit to initiate the charging in response to the first signal, and control the charging unit to allow the electrical shock to be output in response to a second signal to be received through the communication unit.

According to yet another aspect of the present invention, there is provided a mobile terminal controlling a portable defibrillation device, the mobile terminal including a communication unit configured to communicate with the portable defibrillation device, and a control unit configured to select one charging mode from among a predetermined number of charging modes, generate a first signal based on the selected charging mode, and control the communication unit to allow the first signal to be transmitted to the portable defibrillation device.

The predetermined number of the charging modes may include at least one of a first charging mode using a power source of the mobile terminal, a second charging mode using a power source of the portable defibrillation device, and a third charging mode using the power source of the mobile terminal and the power source of the portable defibrillation device.

The control unit may select the charging mode based on at least one of an amount of power remaining in the power source of the mobile terminal, an output characteristic of the power source of the mobile terminal, an amount of power remaining in the power source of the portable defibrillation device, and an output characteristic of the power source of the portable defibrillation device.

The control unit may select the charging mode based on a determination that an electrical shock is required for defibrillation, generate a second signal based on a determination that a condition for the electrical shock is satisfied, and control the communication unit to allow the second signal to be transmitted to the portable defibrillation device.

According to further another aspect of the present invention, there is provided an operating method of a mobile terminal controlling a portable defibrillation device, the method including determining whether an electrical shock is required for defibrillation, generating a first signal based on a determination that the electrical shock is required, determining whether a condition for the electrical shock is satisfied, and generating a second signal based on a determination that the condition for the electrical shock is satisfied.

According to still another aspect of the present invention, there is provided an operating method of a mobile terminal controlling a portable defibrillation device, the method including selecting one charging mode from among a predetermined number of charging modes, and generating a first signal based on the selected charging mode.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a diagram illustrating a mobile terminal and a portable defibrillation device according to an embodiment of the present invention;
FIGS. 2 through 6 are diagrams illustrating examples of generating a control signal according to embodiments of the present invention;
FIG. 7 is a diagram illustrating a power source charging a portable defibrillation device according to an embodiment of the present invention;
FIG. 8 is a diagram illustrating a combination between a mobile terminal and a portable defibrillation device according to an embodiment of the present invention;
FIG. 9 is a diagram illustrating an input and output interface of a mobile terminal according to an embodiment of the present invention;
FIG. 10 is a diagram illustrating an input and output interface of a portable defibrillation device according to an embodiment of the present invention; and
FIGS. 11 and 12 are flowcharts illustrating an operating method of a mobile terminal according to embodiments of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the accompanying drawings, however, the present invention is not limited thereto or restricted thereby.

When it is determined a detailed description related to a related known function or configuration that may make the purpose of the present invention unnecessarily ambiguous in describing the present invention, the detailed description will be omitted here. Also, terms used herein are defined to appropriately describe the exemplary embodiments of the present invention and thus may be changed depending on a user, the intent of an operator, or a custom. Accordingly, the terms must be defined based on the following overall description of this specification.

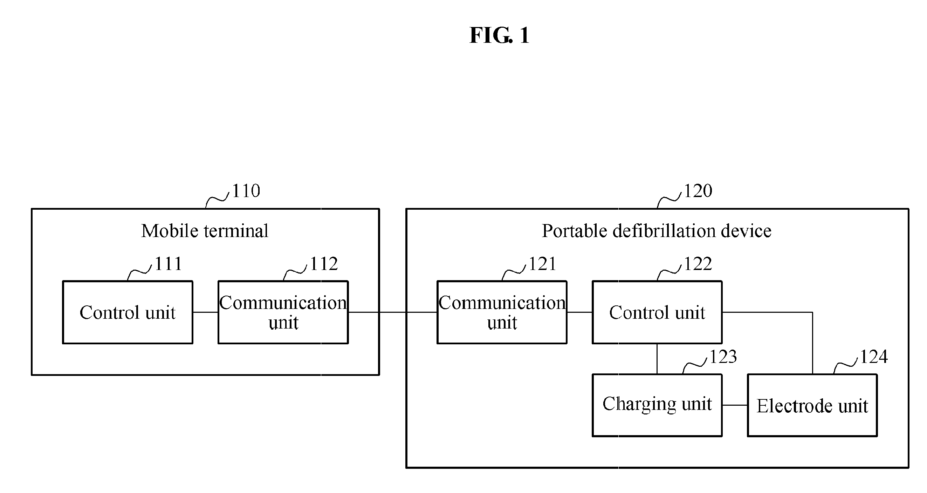

FIG. 1 is a diagram illustrating a mobile terminal 110 and a portable defibrillation device 120 according to an embodiment of the present invention. An automatic defibrillation function may be provided to a user suffering from a cardiac disorder through interworking between the mobile terminal 110 and the portable defibrillation device 120. The mobile terminal 110 may be a portable computing device that may be carried by the user and include, for example, a mobile phone, a smartphone, a tablet computer, a laptop computer, and a wearable device of the user. The portable defibrillation device 120 may be a portable computing device that may be carried by the user and output an electrical shock for defibrillation by interworking with, for example, a mobile phone, a smartphone, a tablet computer, a laptop computer, and a wearable device of the user. The portable defibrillation device 120 may be provided in a form physically combinable to the mobile terminal 110 to allow the user to readily carry both the mobile terminal 110 and the portable defibrillation device 120.

Referring to FIG. 1, the portable defibrillation device 120 includes a communication unit 121, a control unit 122, a charging unit 123, and an electrode unit 124. The communication unit 121 may communicate with the mobile terminal 110. For example, the communication unit 121 may communicate with the mobile terminal 110 using a wired or a wireless communication interface. The communication unit 121 may communicate with the mobile terminal 110 based on a standard for a data communication interface fundamentally loaded in a process of manufacturing the mobile terminal 110.

The charging unit 123 may charge an energy storage for defibrillation. The energy storage may be a device, an element, a material, or a circuit to store electric energy. For example, the energy storage may include a capacitor or a super-capacitor. The charging unit 123 may be electrically connected to the electrode unit 124. The charging unit 123 may charge the energy storage for the defibrillation under a control of the control unit 122, and output stored electrical power to the electrode unit 124. The charging unit 123 may include a charge and discharge circuit and a safety circuit. The charge and discharge circuit may charge and discharge the energy storage for the defibrillation, and the safety circuit may ensure safety of the user and the charge and discharge circuit in a charging and discharging process.

The electrode unit 124 may output an electrical shock using the energy storage. The electrode unit 124 may be provided in a form attachable to the user and include, for example, electrode pads. The electrode unit 124 may be provided in a form of earphones. The earphones may be designed to allow electrical power to flow for the defibrillation. In addition, the earphones may include an attachment or an attachable material to be attached to the user.

The control unit 122 may control the charging unit 123 based on a control signal received through the communication unit 121. The control signal may be generated by the mobile terminal 110, and the portable defibrillation device 120 may be controlled by the mobile terminal 110 based on the control signal.

For example, the control signal may include a first signal to allow the charging to be initiated and a second signal to allow the electrical shock to be output. The control unit 122 may control the charging unit 123 to allow the charging to be initiated in response to the first signal.

The first signal may be generated based on whether the electrical shock is required for the defibrillation. For example, the first signal may be generated by the mobile terminal 110. The mobile terminal 110 may determine whether the electrical shock is required for the defibrillation, and generate the first signal based on a determination that the electrical shock is required for the defibrillation. Here, the mobile terminal 110 may determine whether the electrical shock is required for the defibrillation in accordance with a predetermined process, and automatically generate the first signal without waiting for an input from the user when the electrical shock is determined to be required for the defibrillation. Although further described hereinafter, the mobile terminal 110 may detect the user collapsing using an acceleration sensor, or detect an abnormal state of the user using a biosignal of the user. In such a case, the mobile terminal 110 may determine that the electrical shock is required for the defibrillation and generate the first signal.

The portable defibrillation device 120 may charge the energy storage based on the first signal. When the portable defibrillation device 120 receives the first signal, the portable defibrillation device 120 may charge the energy storage irrespective of whether the electrical shock is actually output for the defibrillation. The portable defibrillation device 120 may charge the energy storage in advance for the defibrillation and thus, may reduce an amount of time necessary for outputting the electrical shock for actual defibrillation.

In addition, the control unit 122 may control the charging unit 123 to output the electrical shock in response to the second signal. The second signal may be generated based on whether a condition for the electrical shock is satisfied. For example, the second signal may be generated by the mobile terminal 110. The mobile terminal 110 may generate the second signal based on a determination that the condition for the electrical shock is satisfied. The mobile terminal 110 may determine whether the condition for the electrical shock is satisfied based on a predetermined process, and generate the second signal when the condition for the electrical shock is determined to be satisfied. Although further described hereinafter, the mobile terminal 110 may determine whether the condition for the electrical shock is satisfied based on, for example, whether the electrode pads included in the electrode unit 124 of the portable defibrillation device 120 are attached to the user and whether the charging is completed by the charging unit 123 of the portable defibrillation device 120.

As illustrated in FIG. 1, the mobile terminal 110 includes a control unit 111 and a communication unit 112. The communication unit 112 may communicate with the portable defibrillation device 120. For example, the communication unit 112 may communicate with the portable defibrillation device 120 using a wired or a wireless communication interface. The communication unit 112 may communicate with the portable defibrillation device 120 based on a standard for a data communication interface fundamentally loaded in a process of manufacturing the mobile terminal 110.

The control unit 111 may generate a control signal to control the portable defibrillation device 120. For example, the control unit 111 may determine whether an electrical shock is required for defibrillation, and generate a first signal based on a determination that the electrical shock is required for the defibrillation. The control unit 111 may control the communication unit 112 to allow the generated first signal to be transmitted to the portable defibrillation device 120. As further described hereinafter, the control unit 111 may detect whether the user collapses using an output of the acceleration sensor, or detect an abnormal state of the user using a biosignal of the user. In such a case, the control unit 111 may determine that the electrical shock is required for the defibrillation and generate the first signal.

In addition, the control unit 111 may determine whether a condition for the electrical shock is satisfied and generate a second signal based on a determination that the condition for the electrical shock is satisfied. The control unit 111 may control the communication unit 112 to allow the generated second signal to be transmitted to the portable defibrillation device 120. As further described hereinafter, the control unit 111 may determine whether the condition for the electrical shock is satisfied based on, for example, whether the electrode pads included in the electrode unit 124 of the portable defibrillation device 120 are attached to the user and whether the charging is completed by the charging unit 123 of the portable defibrillation device 120.

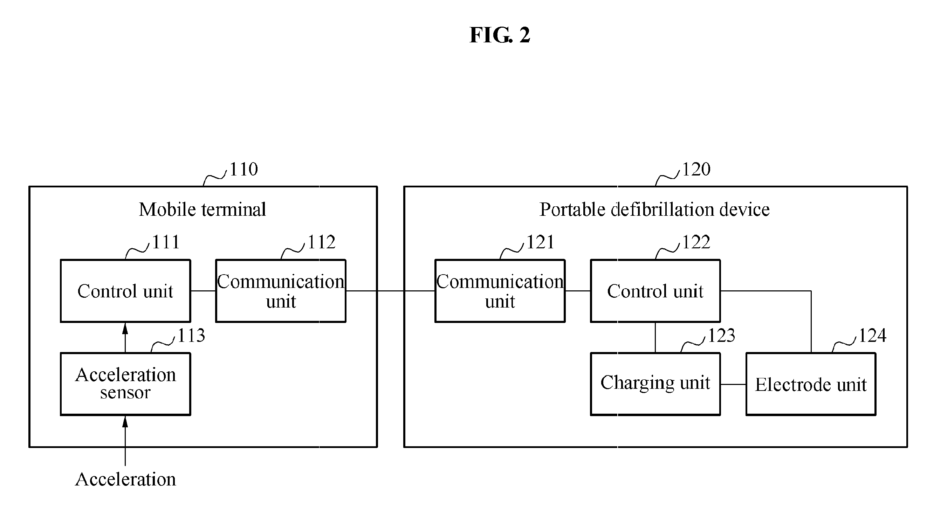

FIGS. 2 through 6 are diagrams illustrating examples of generating a control signal according to embodiments of the present invention. Referring to FIG. 2, a mobile terminal 110 further includes an acceleration sensor 113. The acceleration sensor 113 may measure an acceleration of the mobile terminal 110. A control unit 111 of the mobile terminal 110 may determine whether an electrical shock is required for defibrillation using an output of the acceleration sensor 113.

For example, when a user of the mobile terminal 110 collapses from a cardiac disorder, the control unit 111 may determine whether the output of the acceleration sensor 113 occurs due to the user of the mobile terminal 110 collapsing. When the output of the acceleration sensor 113 is determined to occur due to the user of the mobile terminal 110 collapsing, the control unit 111 may determine that the electrical shock is required for the defibrillation. The control unit 111 may generate a first signal and control a communication unit 112 of the mobile terminal 110 to allow the first signal to be transmitted to a portable defibrillation device 120.

The control unit 111 may determine whether the electrical shock is required for the defibrillation using a user customized parameter. In an example, the control unit 111 may determine whether the output of the acceleration sensor 113 occurs due to the user of the mobile terminal 110 collapsing through a comparison of acceleration information associated with habitual actions of the user to the output of the acceleration sensor 113. For the comparison, the control unit 111 may store the acceleration information associated with the habitual actions of the user in a pre-equipped storage device. In addition, the control unit 111 may periodically update the acceleration information associated with the habitual actions of the user. The pre-equipped storage device may encompass, for example, a memory included in the mobile terminal 110, a memory included in the portable defibrillation device 120, and a server connected through a network.

In another example, the control unit 111 may determine whether the output of the acceleration sensor 113 occurs due to the user of the mobile terminal 110 collapsing through a comparison of a threshold value corresponding to an option set by the user, to the output of the acceleration sensor 113. For the comparison, the control unit 111 may obtain the threshold value corresponding to the option set by the user from the pre-equipped storage device.

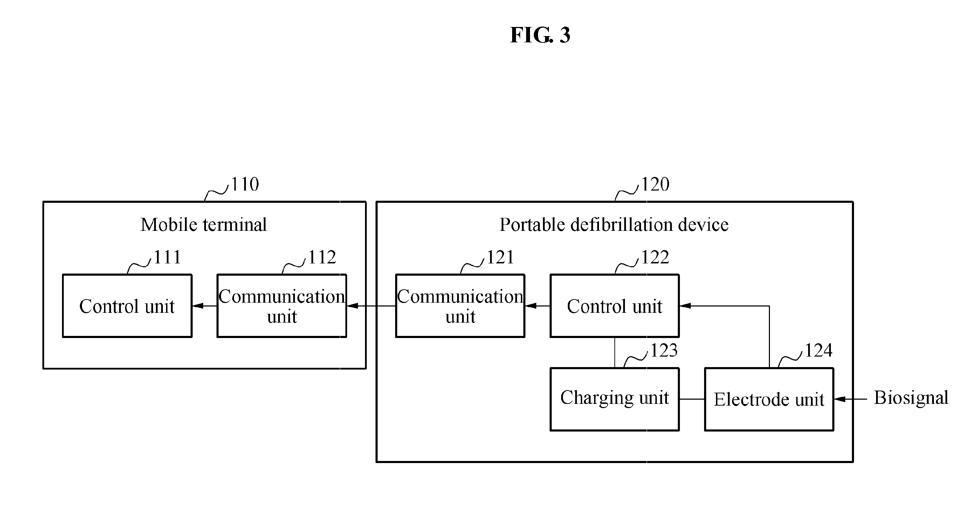

Referring to FIG. 3, a mobile terminal 110 may determine whether an electrical shock is required for defibrillation based on a biosignal to be detected by a portable defibrillation device 120.

For example, electrode pads included in an electrode unit 124 of the portable defibrillation device 120 may be attached to a user. In such an example, a control unit 122 of the portable defibrillation device 120 may detect a biosignal to be received through the electrode unit 124, and control a communication unit 121 of the portable defibrillation device 120 to allow information associated with the detected biosignal to be transmitted to the mobile terminal 110. The information associated with the biosignal may be the detected biosignal itself or a signal obtained from the detected biosignal converted to a predetermined format, depending on a situation.

A communication unit 112 of the mobile terminal 110 may receive the information associated with the biosignal transmitted from the portable defibrillation device 120, and a control unit 111 of the mobile terminal 110 may determine whether the electrical shock is required for the defibrillation based on the information associated with the biosignal. The control unit 111 may detect an abnormal state, for example, an arrhythmia, from the biosignal of the user, or detect a symptom that has a high probability of prompting the abnormal state. In such a case, the control unit 111 may determine that the electrical shock is required for the defibrillation and generate a first signal. The control unit 111 may control the communication unit 112 to allow the first signal to be transmitted to the portable defibrillation device 120.

The control unit 111 may determine whether the electrical shock is required for the defibrillation using a user customized parameter. In an example, the control unit 111 may determine whether a currently detected biosignal is generated due to the abnormal state of the user through a comparison of biosignal information of the user in a usual state to the currently detected biosignal. For the comparison, the control unit 111 may store the biosignal information of the user in the usual state in a pre-equipped storage device. In addition, the control unit 111 may periodically update the biosignal information of the user in the usual state.

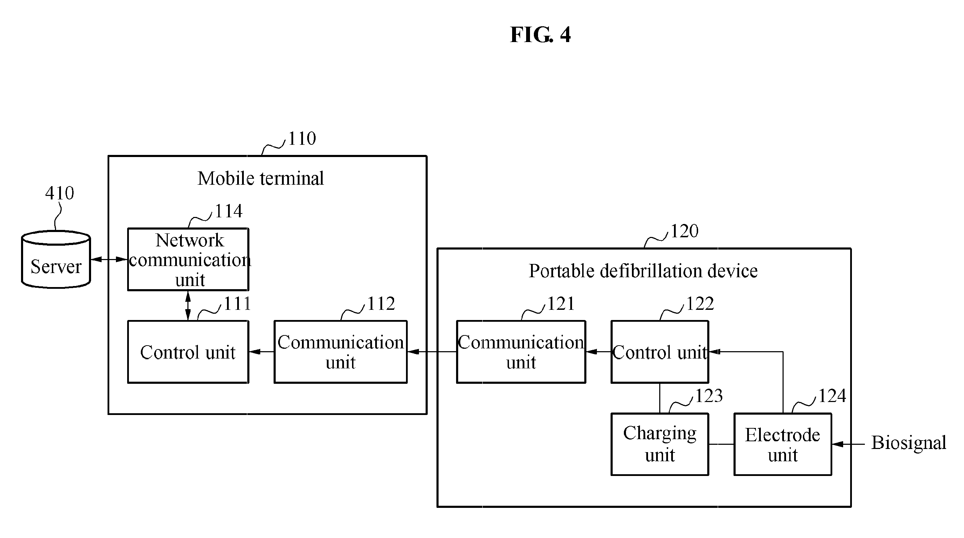

Referring to FIG. 4, a mobile terminal 110 further includes a network communication unit 114. The network communication unit 114 may communicate with a server 410 connected through a network. For example, the network communication unit 114 may communicate with the server 410 using a wired network, for example, a wired local area network (LAN), or a wireless network, for example, a mobile communication network and a wireless LAN.

A control unit 111 of the mobile terminal 110 may control the network communication unit 114 to allow information associated a biosignal received from a portable defibrillation device 120 to be transmitted to the server 410. The server 410 may determine whether an electrical shock is required for defibrillation using the information associated with the biosignal. For example, the server 410 may detect an abnormal state, for example, an arrhythmia, from the biosignal of the user, or detect a symptom that has a high probability of prompting the abnormal state. In such an example, the server 410 may transmit a first response signal to the mobile terminal 110. However, when the electrical shock is not determined to be required for the defibrillation, the server 410 may transmit a second response signal to the mobile terminal 110.

The server 410 may determine whether the electrical shock is required for the defibrillation using a user customized parameter. In an example, the server 410 may determine whether a currently detected biosignal is generated due to the abnormal state of the user by comparing biosignal information of the user in a usual state to the currently detected biosignal. For the comparison, the server 410 may store the biosignal information of the user in the usual state. In addition, the server 410 may periodically update the biosignal information of the user in the usual state.

The control unit 111 may determine whether the electrical shock is required for the defibrillation based on the response signals received through the network communication unit 114. For example, when the first response signal is received, the control unit 111 may determine that the electrical shock is required for the defibrillation. In such an example, the control unit 111 may generate a first signal. Alternatively, the control unit 111 may determine whether a response signal received through the network communication unit 114 is the first response signal, and generate the first signal when the received response signal is the first response signal. The control unit 111 may control a communication unit 112 of the mobile terminal 110 to allow the generated first signal to be transmitted to the portable defibrillation device 120.

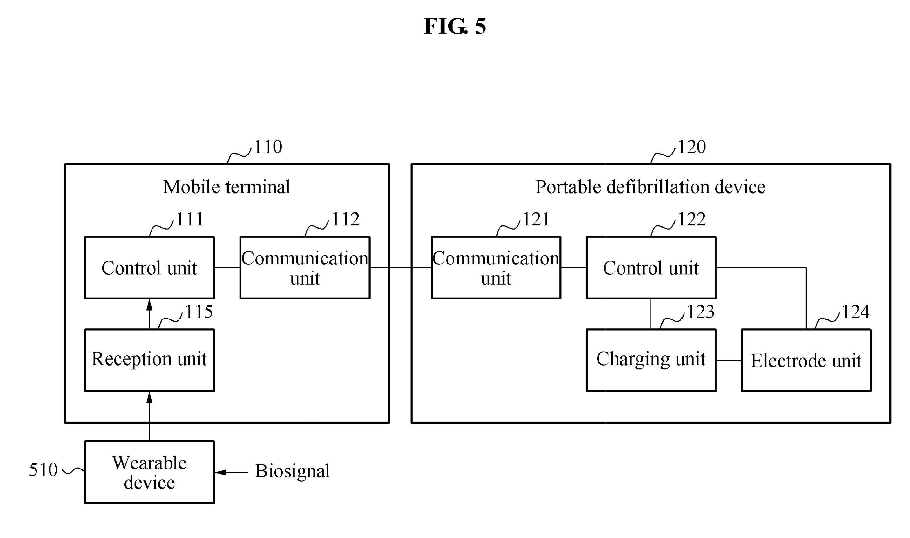

Referring to FIG. 5, a mobile terminal 110 further includes a reception unit 115. The reception unit 115 may communicate with a wearable device 510 using a wired or a wireless interface. For example, the reception unit 115 may communicate with the wearable device 510 using Bluetooth^{™} communication.

The mobile terminal 110 may determine whether an electrical shock is required for defibrillation based on a biosignal detected by the wearable device 510. The wearable device 510 may be a device that may be worn by a user, and include devices provided in various forms, for example, glasses, a watch, fashion accessories, earphones, headphones, a hat or a cap, a helmet, a vest, a belt, a scarf, shoes, a top, bottoms, inner wear, and a coat.

The user may wear the wearable device 510. In such a case, the wearable device 510 may detect a biosignal, and transmit information associated with the detected biosignal to the mobile terminal 110. The information associated with the biosignal may be the detected biosignal itself or a signal obtained from the detected biosignal converted to a predetermined format according to circumstances.

The reception unit 115 may receive the information associated with the biosignal transmitted from the wearable device 510, and a control unit 111 of the mobile terminal 110 may determine whether the electrical shock is required for the defibrillation based on the information associated with the biosignal. The control unit 111 may detect an abnormal state, for example, an arrhythmia, from the biosignal of the user, or detect a symptom that has a high probability of prompting the abnormal state. In such a case, the control unit 111 may determine that the electrical shock is required for the defibrillation and generate a first signal. The control unit 111 may control a communication unit 112 of the mobile terminal 110 to allow the first signal to be transmitted to a portable defibrillation device 120.

The control unit 111 may determine whether the electrical shock is required for the defibrillation using a user customized parameter. In an example, the control unit 111 may determine whether a currently detected biosignal is generated due to the abnormal state of the user through a comparison of biosignal information of the user in a usual state to the currently detected biosignal. For the comparison, the control unit 111 may store the biosignal information of the user in the usual state in a pre-equipped storage device. In addition, the control unit 111 may periodically update the biosignal information of the user in the usual state.

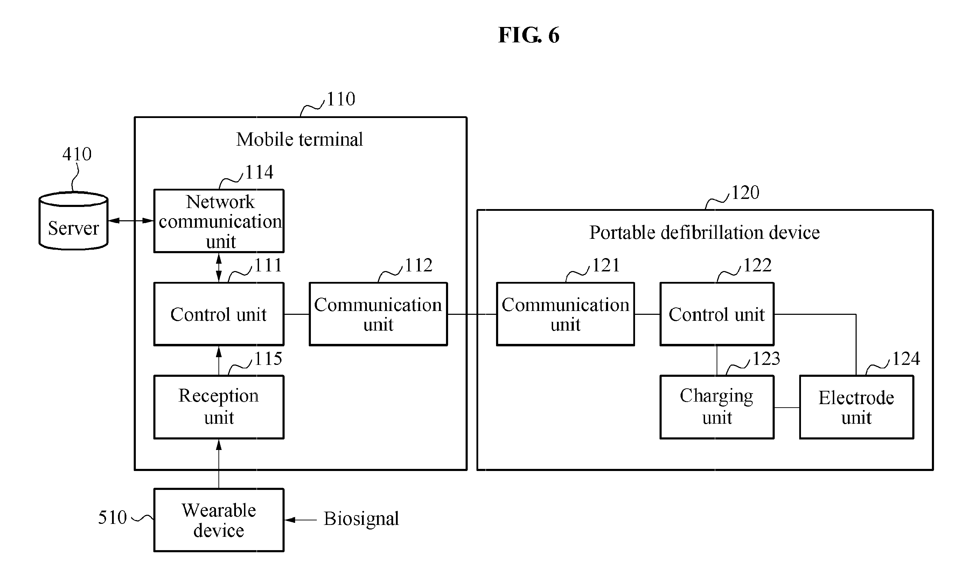

Referring to FIG. 6, a mobile terminal 110 further includes a network communication unit 114. The network communication unit 114 may communicate with a server 410 using a wired network, for example, a wired LAN, or a wireless network, for example, a mobile communication network and a wireless LAN.

A control unit 111 of the mobile terminal 110 may control the network communication unit 114 to allow information associated a biosignal received from a wearable device 510 to be transmitted to the server 410. The server 410 may determine whether an electrical shock is required for defibrillation using the information associated with the biosignal. For example, the server 410 may detect an abnormal state, for example, an arrhythmia, from the biosignal of a user, or detect a symptom that is highly likely to provoke the abnormal state. In such an example, the server 410 may transmit a first response signal to the mobile terminal 110. However, when the electrical shock is not determined to be required for the defibrillation, the server 410 may transmit a second response signal to the mobile terminal 110.

The server 410 may determine whether the electrical shock is required for the defibrillation using a user customized parameter. In an example, the server 410 may determine whether a currently detected biosignal is generated due to the abnormal state of the user through a comparison of biosignal information of the user in a usual state to the currently detected biosignal. For the comparison, the server 410 may store the biosignal information of the user in the usual state. In addition, the server 410 may periodically update the biosignal information of the user in the usual state.

The control unit 111 may determine whether the electrical shock is required for the defibrillation based on the response signals received through the network communication unit 114. For example, when the first response signal is received, the control unit 111 may determine that the electrical shock is required for the defibrillation. In such an example, the control unit 111 may generate a first signal. Alternatively, the control unit 111 may determine whether a response signal received through the network communication unit 114 is the first response signal, and generate the first signal when the received response signal is the first response signal. The control unit 111 may control a communication unit 112 of the mobile terminal 110 to allow the generated first signal to be transmitted to a portable defibrillation device 120.

Referring back to FIG. 1, the mobile terminal 110 may determine whether the condition for the electrical shock is satisfied and generate the second signal based on the determination that the condition for the electrical shock is satisfied.

Various conditions may be applicable to the electrical shock. The condition for the electrical shock may include, for example, a condition that the electrode pads included in the electrode unit 124 of the portable defibrillation device 120 are attached to the user, a condition that the charging is completed by the charging unit 123 of the portable defibrillation device 120, and a condition that a timing at which the electrical shock is applied to the user is satisfied. Alternatively, the condition for the electrical shock may include a condition that other people are not in contact with the user for safety of others present in an adjacent area. The condition for the electrical shock may be configured as a single condition of the conditions described in the foregoing or at least two conditions combined, as necessary.

In an example, the portable defibrillation device 120 may provide the mobile terminal 110 with information as to whether the electrode pads included in the electrode unit 124 are optimally attached to the user. The portable defibrillation device 120 may determine whether the electrode pads are optimally attached to the user using a signal received by the electrode unit 124, and provide the mobile terminal 110 with the information including a result of the determining. Alternatively, the portable defibrillation device 120 may provide the mobile terminal 110 with the signal received by the electrode unit 124 to allow the mobile terminal 110 to determine whether the electrode pads are optimally attached to the user.

In another example, the portable defibrillation device 120 may provide the mobile terminal 110 with information associated with a biosignal received by the electrode unit 124, and the mobile terminal 110 may calculate a timing for the electrical shock. The mobile terminal 110 may analyze the biosignal of the user and calculate an appropriate timing at which the electrical shock is applied to the user. The mobile terminal 110 may generate the second signal based on the calculated timing and transmit the generated second signal to the portable defibrillation device 120. In response to the second signal, the portable defibrillation device 120 may output the electrical shock through the electrode unit 124.

Alternatively, the mobile terminal 110 may generate the second signal including timing information on the calculated timing and transmit the generated second signal to the portable defibrillation device 120. The portable defibrillation device 120 may output the electrical shock through the electrode unit 124 in accordance with a timing corresponding to the timing information included in the second signal.

In still another example, the portable defibrillation device 120 may provide the mobile terminal 110 with information as to whether the charging is completed by the charging unit 123. The portable defibrillation device 120 may determine whether the charging is completed based on an amount of electrical power stored in the energy storage by the charging unit 123, and provide the mobile terminal 110 with the information, including a result of the determining. Alternatively, the portable defibrillation device 120 may provide the mobile terminal 110 with the amount of the electrical power stored in the energy storage by the charging unit 123 to allow the mobile terminal 110 to determine whether the charging is completed.

The control unit 111 may generate the second signal based on the determination that the condition for the electrical shock is satisfied, and control the communication unit 112 to allow the generated second signal to be transmitted to the portable defibrillation device 120.

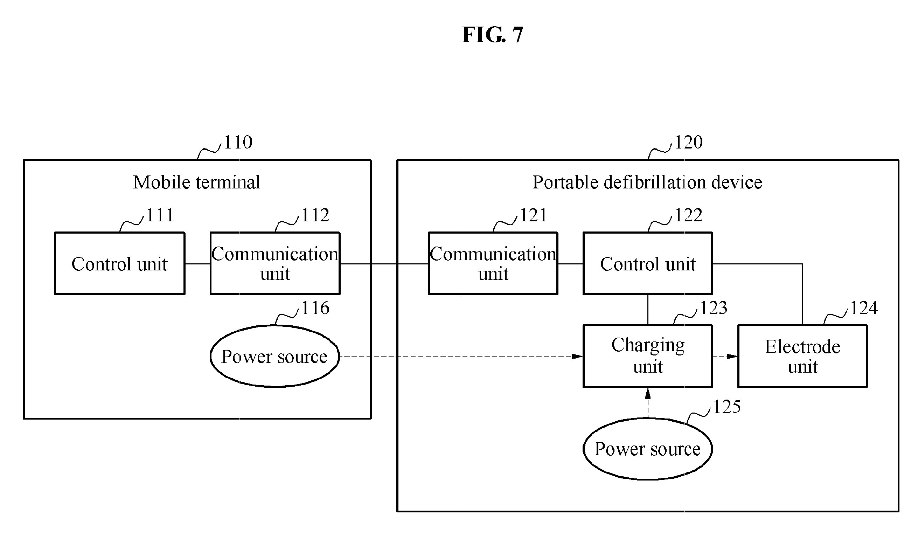

FIG. 7 is a diagram illustrating a power source 125 charging a portable defibrillation device 120 according to an embodiment of the present invention. Referring to FIG. 7, a charging unit 123 of the portable defibrillation device 120 is electrically connected to a power source 116 of a mobile terminal 110. Thus, the charging unit 123 may charge an energy storage for defibrillation using the power source 116 of the mobile terminal 123.

According to another embodiment, the portable defibrillation device 120 may further include the power source 125. Thus, the charging unit 123 may charge the energy storage for the defibrillation using the power source 125.

According to still another embodiment, the charging unit 123 may be electrically connected to the power source 116, and the portable defibrillation device 120 may further include the power source 125. Thus, the charging unit 123 may charge the energy storage for the defibrillation using at least one of the power source 116 and the power source 125.

The mobile terminal 110 may select one charging mode from a plurality of predetermined charging modes. The predetermined charging modes may include at least one of a first charging mode using the power source 116, a second charging mode using the power source 125, and a third charging mode using the power source 116 and the power source 125.

The charging mode may be selected based on at least one of an amount of power remaining in the power source 116, an output characteristic of the power source 116, an amount of power remaining in the power source 125, and an output characteristic of the power source 125. Various selection scenarios may be applicable to the charging mode.

In an example, the mobile terminal 110 may preferentially select the second charging mode using the power source 125 of the portable defibrillation device 120. The mobile terminal 110 may compare the amount of power remaining in the power source 125 to a predetermined threshold value. When the amount of power remaining in the power source 125 is greater than or equal to the predetermined threshold value, the mobile terminal 110 may select the second charging mode. When the amount of power remaining in the power source 125 is less than the predetermined threshold value, the mobile terminal 110 may select the first charging mode or the third charging mode. The mobile terminal 110 may update the selected charging mode by periodically inspecting the amount of power remaining in the power source 125.

Alternatively, the mobile terminal 110 may evaluate the output characteristic of the power source 125. When the output characteristic of the power source 125 is greater than or equal to a predetermined threshold value, the mobile terminal 110 may select the second charging mode. When the output characteristic of the power source 125 is less than the predetermined threshold value, the mobile terminal 110 may select the first charging mode or the third charging mode. The mobile terminal 110 may update the selected charging mode by periodically evaluating the output characteristic of the power source 125.

In another example, the mobile terminal 110 may preferentially select the first charging mode using the power source 116 of the mobile terminal 110. The mobile terminal 110 may compare the amount of power remaining in the power source 116 to a predetermined threshold value. When the amount of power remaining in the power source 116 is greater than or equal to the predetermined threshold value, the mobile terminal 110 may select the first charging mode. When the amount of power remaining in the power source 116 is less than the predetermined threshold value, the mobile terminal 110 may select the second charging mode or the third charging mode. The mobile terminal 110 may update the selected charging mode by periodically inspecting the amount of power remaining in the power source 116.

Alternatively, the mobile terminal 110 may evaluate the output characteristic of the power source 116. When the output characteristic of the power source 116 is greater than or equal to a predetermined threshold value, the mobile terminal 110 may select the first charging mode. When the output characteristic of the power source 116 is less than the predetermined threshold value, the mobile terminal 110 may select the second charging mode or the third charging mode. The mobile terminal 110 may update the selected charging mode by periodically evaluating the output characteristic of the power source 116.

In still another example, the mobile terminal 110 may select a charging mode in which the energy storage may be charged fastest for the defibrillation. The mobile terminal 110 may calculate a charging time for each charging mode based on the amount of power remaining in the power source 116 and the amount of power remaining in the power source 125. The mobile terminal 110 may further calculate the charging time for each charging mode based on a prestored output characteristic of the power source 116 and a prestored output characteristic of the power source 125. Alternatively, the mobile terminal 110 may evaluate the output characteristic of the power source 116 and the output characteristic of the power source 125, and further calculate the charging time for each charging mode based on the evaluated output characteristic of the power source 116 and the evaluated output characteristic of the power source 125. The mobile terminal 110 may select a charging mode having a smallest charging time.

The mobile terminal 110 may include, in the first signal, information indicating the selected charging mode. The portable defibrillation device 120 may receive the first signal from the mobile terminal 110, and charge the charging unit 123 based on the charging mode indicated in the information included in the first signal.

For example, the control unit 122 of the portable defibrillation device 120 may control the charging unit 123 to initiate the charging of the energy storage in response to the first signal. When the information included in the first signal indicates the first charging mode, the control unit 122 may control the charging unit 123 to perform the charging using the power source 116. When the information included in the first signal indicates the second charging mode, the control unit 122 may control the charging unit 123 to perform the charging using the power source 125. When the information included in the first signal indicates the third charging mode, the control unit 122 may control the charging unit 123 to perform the charging using the power source 116 and the power source 125.

When the power source 125 is included in the portable defibrillation device 120, the power source 125 may be used as an auxiliary battery for the mobile terminal 110. Thus, when the amount of power remaining in the power source 116 is insufficient, the power source 125 may provide electrical power to the mobile terminal 110.

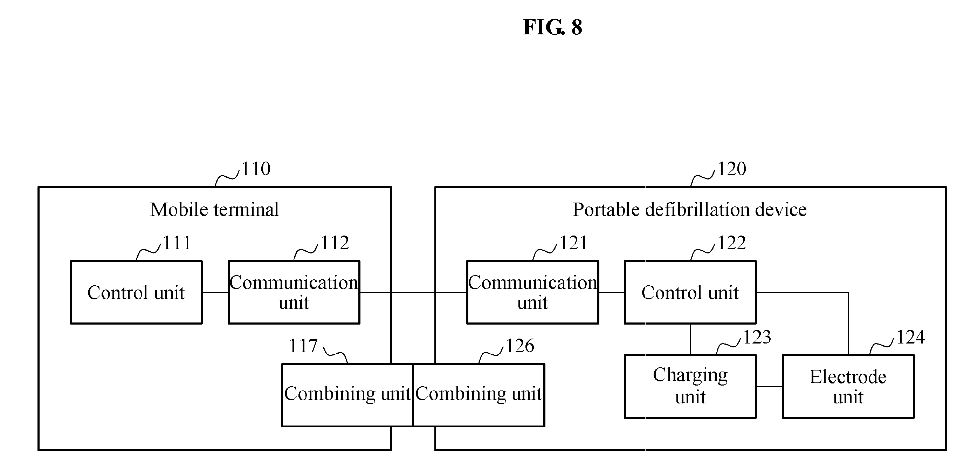

FIG. 8 is a diagram illustrating a combination between a mobile terminal 110 and a portable defibrillation device 120 according to an embodiment of the present invention. Referring to FIG. 8, the mobile terminal 110 and the portable defibrillation device 120 may be combined. A combining unit 117 included in the mobile terminal 110 and a combining unit 126 included in the portable defibrillation device 120 may be designed to be combined to each other. For example, the combining unit 117 and the combining unit 126 may be combined to each other by an external structure, magnetism, and the like.

Example embodiments described with reference to FIGS. 1 through 8 are provided as illustrative examples, and may be variously combined or modified. For example, the mobile terminal 110 may determine whether an electrical shock is required for defibrillation using an output of the acceleration sensor 113 and an output of the wearable device 510 by combining example embodiments described with reference to FIGS. 2 and 5. Example embodiments may be applied to make other combinations.

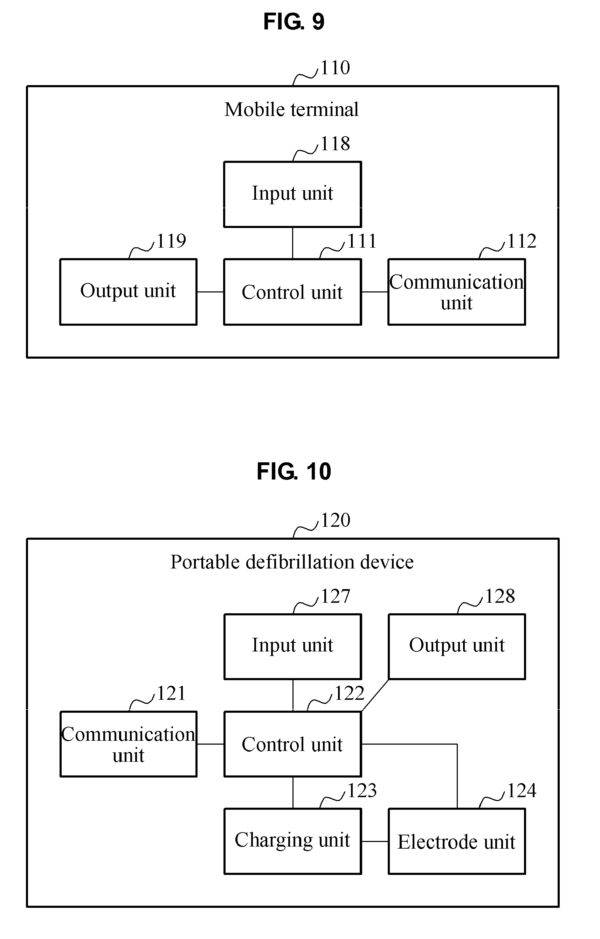

FIG. 9 is a diagram illustrating an input and output interface of a mobile terminal 110 according to an embodiment of the present invention. Referring to FIG. 9, the mobile terminal 110 further includes an output unit 119. The output unit 119 may include a display and a speaker to inform people present around a user of an emergency situation or instruct a defibrillation procedure. Alternatively, the output unit 119 may include an emergency communication unit to inform an emergency center of an emergency situation.

The mobile terminal 110 further includes an input unit 118. The input unit 118 may provide an interface receiving a required input from the user or people present around the user.

FIG. 10 is a diagram illustrating an input and output interface of a portable defibrillation device 120 according to an embodiment of the present invention. Referring to FIG. 10, the portable defibrillation device 120 further includes an output unit 128. The output unit 128 may include a display and a speaker to inform those present around a user of an emergency situation or instruct a defibrillation procedure. Alternatively, the output unit 128 may include an emergency communication unit to inform an emergency center of an emergency situation.

The portable defibrillation device 120 further includes an input unit 127. The input unit 127 may provide an interface receiving a required input from the user or people present around the user.

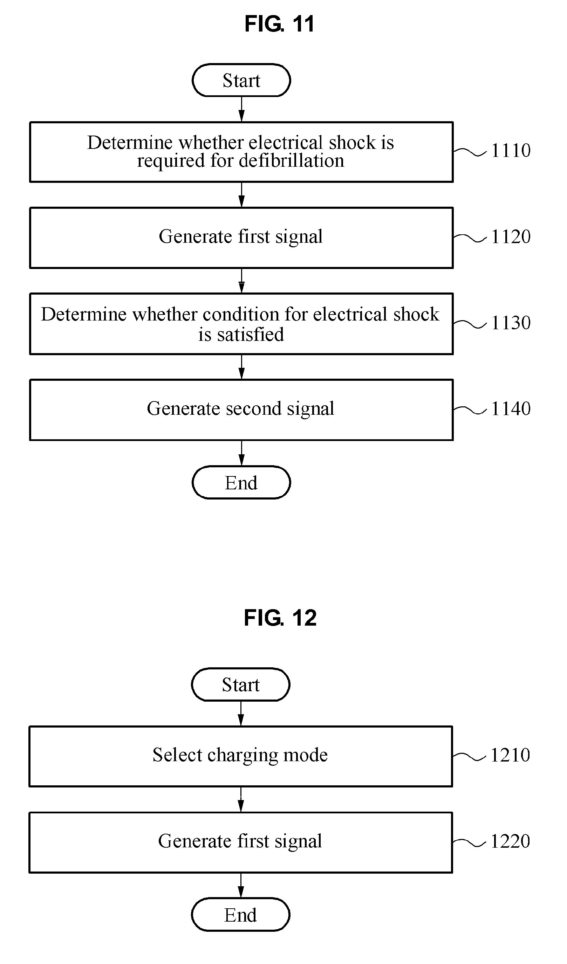

FIGS. 11 and 12 are flowcharts illustrating examples of an operating method of a mobile terminal according to embodiments of the present invention. Referring to FIG. 11, an operating method of a mobile terminal controlling a portable defibrillation device includes operation 1110 of determining whether an electrical shock is required for defibrillation, operation 1120 of generating a first signal based on a determination that the electrical shock is required, operation 1130 of determining whether a condition for the electrical shock is satisfied, and operation 1140 of generating a second signal based on a determination that the condition for the electrical shock is satisfied.

Referring to FIG. 12, an operating method of a mobile terminal controlling a portable defibrillation device includes operation 1210 of selecting a predetermined charging mode and operation 1220 of generating a first signal based on the selected charging mode.

Technical descriptions provided with reference to FIGS. 1 through 7 may be applicable to the operations described with reference to FIGS. 11 and 12 and thus, a repeated description will be omitted here for brevity.

The units described herein may be implemented using hardware components and software components. For example, the hardware components may include microphones, amplifiers, band-pass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

The above-described example embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM discs and DVDs; magneto-optical media such as floptical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described example embodiments of the present invention, or vice versa.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. A portable defibrillation device, comprising:
a communication unit configured to communicate with a mobile terminal;
a charging unit configured to charge an energy storage for defibrillation;
an electrode unit configured to output an electrical shock using the energy storage;
a control unit configured to control the charging unit to initiate the charging of the energy storage in response to a first signal received through the communication unit, and control the charging unit to allow the electrical shock to be output in response to a second signal received through the communication unit; and
a combining unit combined to the mobile terminal, and
wherein the first signal is automatically generated based on whether the electrical shock is required for the defibrillation.

2. The device of claim 1, wherein the control unit is configured to control the communication unit to allow a biosignal detected through the electrode unit to be transmitted to the mobile terminal, and
at least one of the first signal and the second signal is generated based on the biosignal.

3. The device of claim 1 or 2, wherein the first signal is generated based on at least one of an output of an acceleration sensor comprised in the mobile terminal and an output of a wearable device worn by a user.

4. The device of any one of claims 1-3, wherein the charging unit is configured to charge the energy storage using a power source of the mobile terminal.

5. The device of any one of claims 1-4, further comprising:
a power source electrically connected to the charging unit, and
wherein the control unit is configured to control the charging unit to charge the energy storage using at least one of the power source of the portable defibrillation device and a power source of the mobile terminal based on the first signal,
wherein the first signal is preferably further generated based on at least one of an amount of power remaining in the power source of the mobile terminal, an output characteristic of the power source of the mobile terminal, an amount of power remaining in the power source of the portable defibrillation device, and an output characteristic of the power source of the portable defibrillation device,
wherein the control unit is preferably configured to control the charging unit to charge the energy storage using the power source of the mobile terminal when information comprised in the first signal indicates a first charging mode,
the control unit is configured to control the charging unit to charge the energy storage using the power source of the portable defibrillation device when the information comprised in the first signal indicates a second charging mode, and
the control unit is configured to control the charging unit to charge the energy storage using the power source of the portable defibrillation device and the power source of the mobile terminal when the information comprised in the first signal indicates a third charging mode, and/or
wherein the power source of the portable defibrillation device is preferably configured to supply auxiliary power to the mobile terminal.

6. A mobile terminal, comprising:
a communication unit configured to communicate with a portable defibrillation device; and
a control unit configured to generate a first signal indicating initiation of charging an energy storage based on a determination that an electrical shock is required for defibrillation, control the communication unit to allow the first signal to be transmitted to the portable defibrillation device, generate a second signal indicating an output of the electrical shock from the energy storage based on a determination that a condition for the electrical shock is satisfied, and control the communication unit to allow the second signal to be transmitted to the portable defibrillation device.

7. The mobile terminal of claim 6, wherein the communication unit is configured to receive information associated with a biosignal of a user, and
the control unit is configured to determine whether the electrical shock is required for the defibrillation based on the information associated with the biosignal of the user.

8. The mobile terminal of claim 6 or 7, further comprising:
a reception unit configured to receive information associated with a biosignal of a user from a wearable device worn by the user, and
wherein the control unit is configured to determine whether the electrical shock is required for the defibrillation based on the information associated with the biosignal of the user.

9. The mobile terminal of any one of claims 6-8, further comprising:
a network communication unit configured to communicate with a server connected through a network, and
wherein the control unit is configured to control the network communication unit to allow information associated with a biosignal of a user to be transmitted to the server, and determine whether the electrical shock is required for the defibrillation based on a signal received through the network communication unit.

10. The mobile terminal of any one of claims 6-9, further comprising:
an acceleration sensor configured to measure an acceleration of the mobile terminal, and
wherein the control unit is configured to determine whether the electrical shock is required for the defibrillation based on an output of the acceleration sensor.

11. The mobile terminal of any one of claims 6-10, wherein the communication unit is configured to receive information as to whether electrode pads comprised in the portable defibrillation device are attached to a user, and
the control unit is configured to determine whether the condition for the electrical shock is satisfied based on the information as to whether the electrode pads are attached to the user.

12. The mobile terminal of any one of claims 6-11, wherein the communication unit is configured to receive information associated with a biosignal detected by electrode pads comprised in the portable defibrillation device, and
the control unit is configured to calculate a timing for the electrical shock based on the information associated with the biosignal.

13. The mobile terminal of any one of claims 6-12, wherein the control unit is configured to determine a charging mode of the energy storage for the defibrillation based on at least one of an amount of power remaining in a power source of the mobile terminal, an output characteristic of the power source of the mobile terminal, an amount of power remaining in a power source of the portable defibrillation device, and an output characteristic of the power source of the portable defibrillation device,
wherein the charging mode of the energy storage preferably comprises:
a first charging mode using the power source of the mobile terminal;
a second charging mode using the power source of the portable defibrillation device; and
a third charging mode using the power source of the mobile terminal and the power source of the portable defibrillation device.

14. The mobile terminal of any one of claims 6-13, further comprising:
a combining unit combined to the portable defibrillation device.

15. An operating method of a mobile terminal controlling a portable defibrillation device, the method comprising:
determining whether an electrical shock is required for defibrillation;
generating a first signal indicating initiation of charging an energy storage based on a determination that the electrical shock is required;
determining whether a condition for the electrical shock is satisfied; and
generating a second signal indicating an output of the electrical shock from the energy storage based on a determination that the condition for the electrical shock is satisfied.
